# EUROPEAN PATENT SPECIFICATION

(11) **EP 2 424 851 B1**
(45) Date of publication and mention of the grant of the patent: **17.04.2013**
(21) Application number: 10725849.3
(22) Date of filing: 27.04.2010
(51) Int. Cl.: C07D 309/10

(54) **METHODS FOR THE SYNTHESIS OF AMRUBICIN**
VERFAHREN ZUR SYNTHESE VON AMRUBICIN
PROCÉDÉS POUR LA SYNTHÈSE D'AMRUBICINE

(30) Priority: 28.04.2009 US 173440 P
(43) Date of publication of application: 07.03.2012
(73) Proprietor: TBD-BioDiscovery, 50410 Tartu (EE)
(72) Inventor: TSUBRIK, Olga, EE-51013 (EE); TASA, Andrus, Tartumaa (EE); UUSTARE, Ain, EE-50303 Tartu (EE); RINKEN, Ago, EE-51011 Tartu (EE); MÄEORG, Uno, EE-60534 Tartumaa (EE); YLIHONKO, Kristiina, FIN-20760 Piispanristi (FI); HOLMBÄCK, Maria, FIN-21500 Piikkiö (FI); RAUNIO, Jukka, FIN-20760 Piispanristi (FI)
(74) Representative: Rees, Simon John Lewis
(86) International application number: PCT/IB2010/001049
(87) International publication number: WO 2010/125466

(56) References cited:
- WO-A2-01/87814
- US-A1- 2006 047 108
- ISHIZUMI K ET AL: "STEREOSPECIFIC TOTAL SYNTHESIS OF 9-AMINOANTHRACYCLINES: (+)-9AMINO-9-DEOXYDAUNOMYCIN AND RELATED COMPOUNDS" JOURNAL OF ORGANIC CHEMISTRY, AMERICAN CHEMICAL SOCIETY, EASTON.; US LNKD- DOI:10.1021/JO00229A010, vol. 52, no. 20, 1 January 1987 (1987-01-01), pages 4477-4485, XP001179494 ISSN: 0022-3263 cited in the application

## Description

### CROSS-REFERENCE TO RELATED APPLICATION

This application claims the benefit under 35 U.S.C. § 119(e) of the earlier filing date of U.S. Provisional Application Serial Number 61/173,440 filed on April 28, 2009.

### BACKGROUND OF THE INVENTION

### 1. Field of the invention

The present invention relates generally to the novel process to produce a cytotoxic product, amrubicin, which is useful in cancer treatment. The process described herein is also useful to create novel 4-deoxy-9-amino anthracyclines for drug development purposes.

### 2. Description of the background

Amrubicin (Formula I) is an anthracycline antibiotic currently under investigation in the United States for the treatment of small cell lung cancer and in market in Japan. Amrubicin (RN: 110267-81-7) provides some improved aspects in cancer chemotherapy such as reduced dose-limiting cardio toxicity and efficiency in lung cancer (see ref 1 and the references therein). Total chemical synthesis of amrubicin has been described in publication by Ishizumi (J. Org. Chem., 1987, 52, 4477) and respective patent (US 4673668). Due to its large, multi-ring structure and numerous side groups, the synthesis of amrubicin is complicated and requires multiple purification steps. In addition, the prior art method for synthesis of amrubicin and its structural analogs employ large amounts of potassium cyanide and barium salts, both of which are highly toxic.

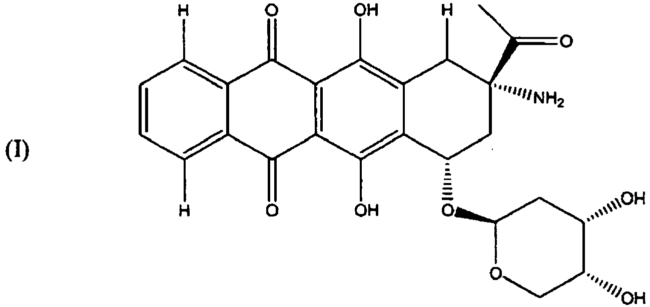

Furthermore, care should be taken during synthesis to maintain the stereochemistry at the multiple chiral centers of the molecule. If stereochemistry of the products is not maintained during synthesis, then costly and laborious separation of enantiomers would be required to obtain the biologically active amrubicin enantiomer.

Semi-synthesis is a type of chemical synthesis that employs compounds produced by natural sources as starting materials. The naturally produced starting material is often complex itself and allows the final product to be synthesized in fewer steps than through traditional chemical synthesis methods. Additionally, biosynthetic steps, i.e. processes that utilize enzyme activities to accomplish chemical transformations, can be employed to achieve efficient synthesis of structurally complex organic compounds with very high enantiomeric purity.

The present invention improves upon the prior art synthetic schemes for amrubicin by combining biosynthetic preparation of the starting material, and traditional chemical approaches to arrive at a safe and efficient method for the production of amrubicin and its structural analogs.

### SUMMARY OF THE INVENTION

The present invention encompasses synthetic pathways for the production of amrubicin (Formula I) and structural analogs thereof. The synthetic pathways of the present invention preferably employ as a starting material an anthracycline compound having the generic Formula II:

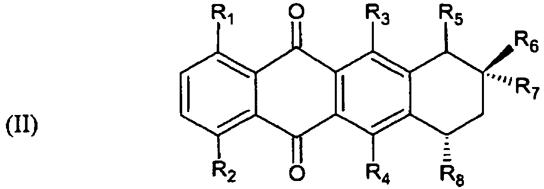

Compounds of Formula II may have any combination of the following identities for the indicated moieties: R₁, R₂, R₃, R₄, and R₈ may be -H, -OH, or alkoxy; R₅ may be -H, alkyl, or alkoxycarbonyl; R₆ may be -H, alkyl or acyl; R₇ is -OH. Compounds of Formula II can be chemically modified to yield aglycone of amrubicin (Formula III).

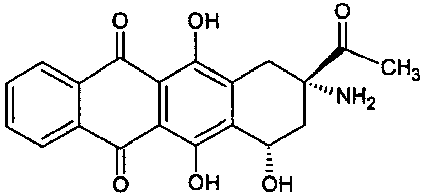

or its structural analogs, which can be chemically glycosylated with respective sugar moiety to form amrubicin (or respective structural analogs thereof). Glycosylation of compound with formula III and its analogs has been described by Ishizumi (J. Org. Chem., 1987, 52, 4477), which is hereby incorporated by reference.

In certain presently preferred embodiments, the starting anthracycline may be ε-rhodomycinone, having the Formula IV:

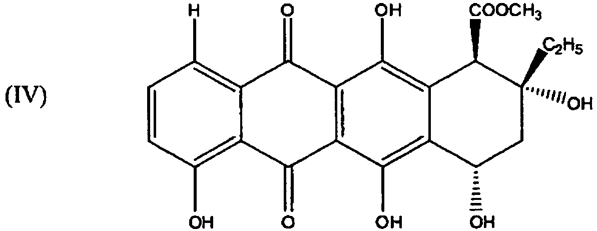

In other presently preferred embodiments, the starting anthracycline may be daunomycinone, having the Formula V:

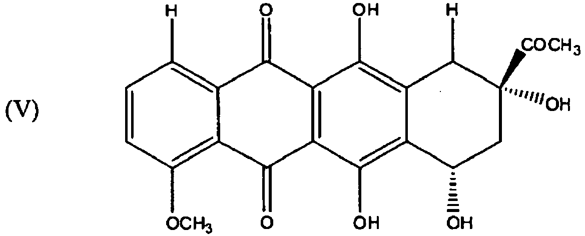

In other presently preferred embodiments, the starting anthracycline may be 4-demethoxy-daunomycinone (idarubicinone) having the Formula VI

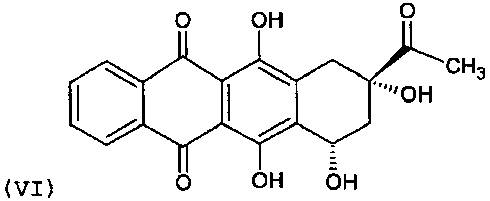

The present invention preferably employs a compound of Formula II as biosynthetically prepared starting material of a semi-synthetic method that combines traditional chemical synthetic steps with biosynthetic starting material to produce amrubicin, derivatives thereof, and structural analogs thereof. The enantiomeric purity of the product is achieved in part by biosynthetic production of the starting compound (Formula II). The particular chemical moieties that are chosen for groups R₁ through R₇ will determine whether the product is amrubicin aglycon or a structural analog thereof.

While synthesis of starting compounds with formula II as defined above is known in the art, biochemical synthesis of respective antracycline analogs where R₇ is - NH₂ (as in aglycon of amrubicine) or modification of biochemically synthesized compounds with formula II to R₇ is -NH₂ is not known by the state of art. Antracycline compounds analogous to formula II with R₇ is NH₂ have been only synthesized by total chemical synthesis which is a difficult multi-step process.

The goal of the invention was therefore to provide methods for chemical modification of compounds with formula II where R₇ is -OH to analogues where R₇ is NH₂, while maintaining the configuration of all chiral carbon atoms in the molecule.

The goal is achieved through subjecting compounds with formula II to a Ritter reaction with a suitable nitrile to form a cyclic intermediate, which is then hydrolyzed in acidic conditions to yield respective derivates with R₇ = -NH₂.

The starting compound according to Formula II is preferably subjected to chemical reactions modifying the substituent R₇ from -OH to -NH₂ to achieve synthesis of the aglycons of amrubicin or structural analogs thereof while maintaining appropriate chirality. Finally, the aglycons of amrubicin or structural analogs thereof may be converted into amrubicin or structural analogs thereof through a chemical glycosylation.

The present invention provides many benefits over the prior art. Generally, the reactions of the present invention are performed under mild conditions, resulting in reduced production of dangerous wastes. The enantiomeric purity of the aglycon molecule is achieved by the biosynthesis of the starting compound. Therefore, none of the following chemical tools are required - asymmetric catalysis, asymmetric synthesis, nor separation of enantiomers of the product.

In contrast to total chemical synthesis of amrubicin and its structural analogs in the prior art where large amounts of potassium cyanide and barium salts are consumed, no such toxic or dangerous reagents are required here during the synthesis. The present invention further avoids photochemical reactions and therefore avoids using special apparatuses employed for such reactions.

The present invention also provides synthetic methods for production of amrubicin aglycone and its structural analogs that require less time and resources, and is therefore considerably more cost-effective and environmentally friendly. Smaller number of chemical steps, smaller amounts of dangerous wastes, and no requirement for the use of expensive equipment make the commercial preparation of amrubicin aglycone and therefore also amrubicin and its structural analogs more efficient and less expensive. All these factors increase the availability of this potent drug for cancer patients.

### DETAILED DESCRIPTION OF THE INVENTION

It is to be understood that the description of the present invention has been simplified to illustrate elements that are relevant for a clear understanding of the invention, while eliminating, for purposes of clarity, other elements that may be well known.

The present invention provides methods for the synthesis of amrubicin and structural analogs thereof. The synthesis preferably begins with an anthracycline starting compound that has been produced through fermentation or semi-synthetic processes. The compound has the formula:

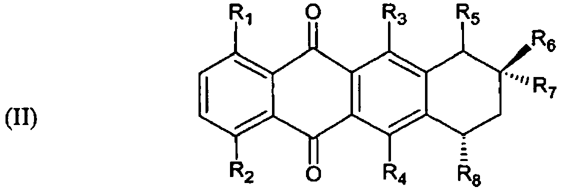

The compound shown in Formula II may include any combination of the following identities for the indicated moieties: R₁, R₂, R₃, R₄, and R₈ may be H, OH, or alkoxy; R₅ may be H, alkyl, or alkoxycarbonyl; R₆ may be -H, alkyl or acyl; R₇ is-OH. Compounds according to Formula II may be synthesized biochemically according to well-known processes. *See, e.g.,* U.S. Patent no. 5,986,077, which is hereby incorporated by reference in its entirety. Daunomycinone, where R₁ is -H, R₂ is -OCH₃, wherein R₃ is -OH, wherein R₄ is -OH, wherein R₅ is -H, wherein R₆ is -COCH₃, wherein R₇ is -OH, and R₈ is OH (Formula V) can be further modified to 4-demethoxydaunomycinone (idarubicinone), where R₁ is -H, R₂ is -OCH₃, wherein R₃ is -OH, wherein R₄ is -OH, wherein R₅ is -H, wherein R₆ is -COCH₃, wherein R₇ is -OH, and R₈ is - OH (Formula VI), as described in WO 01/87814 and US 2006/0047108.

Idarubicinone may be directly aminated to aglycone of amrubicin by employing methods of the present invention.

While the synthetic steps taken from the compound having Formula II to amrubicin or the structural analog of amrubicin will depend on the specific character of each of the moieties, one of skill in the art will recognize that multiple pathways are available within the scope of the present invention. In general, the starting compound of Formula II is modified through a series of chemical reactions to generate aglycons of amrubicin and structural analogs thereof.

The method of choice for amination of antracyclines with Formula II, preferably antracyclines with formula II where R₇ = -OH and R₈ = -OH, in the present invention is Ritter reaction of said compounds with suitable nitriles to get cyclic intermediate with formula VII.

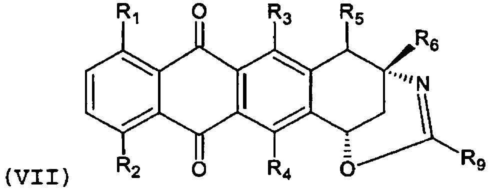

Or a non-cyclic amide intermediate with the formula VIIa

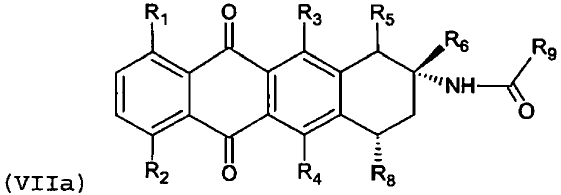

, where R₁-R₆ are as defined above for formula II and R₉ is alkyl, halogenated alkyl, hydrogen or aryl group depending on the used nitrile, followed by hydrolysis of said intermediates with formula VII in acidic conditions.

Hydrolysis of compounds with formula VII is preferably carried out with aqueous solution of a strong acid, preferably strong inorganic acid, most preferably sulfuric acid. Organic solvents, for example tetrahydrofurane, can be added to hydrolysis reaction mixture to improve solubility of anthracyclines and also to facilitate separation of reaction products by extraction.

Other chemical methods like Mitsunobu reaction, or tosylation with the subsequent Gabriel reaction or other chemical amination methods can theoretically also be used for replacement of hydroxyl group with amino group, but most of them require protection of other hydroxyl groups and other functional groups in the molecule. Also, formation of cyclic intermediate with formula VII in the Ritter reaction helps to maintain the enantiomeric purity of the aglycon.

The aglycon product is then preferably glycosylated by 6-bromotetrahydro-2*H-*pyran-3,4-diyl diacetate or other protected derivatives of 2-deoxy-D-ribose diacetyl-ribosylbromide and then deprotected, providing amrubicin (ref. Ishizumi (J. Org. Chem., 1987, 52, 4477)).

### Examples

To more clearly demonstrate the present invention, examples are provided herein below. These are not meant to be limiting, but are instead exemplary embodiments of the general approaches within the scope of the present invention. The yields in the below examples are not optimized. Optimization would involve evaluating multiple parameters, including reactor design and recovery approaches according to processes well known to those of skill in the art. While such efforts would improve the yield, that optimization does not impact the fundamental elements of the disclosed inventive process.

### Example 1

In the present example, the R₇ in 4-dehydroxy-ε-rhodomycinone (R₁ = R₂ = H, R₃ = R₄ = R₈ = OH, R₇ = OH, R₆ = C₂H₅, R₅ = COOCH₃) is transformed into -NH₂ by Ritter amination.

**Step 1: Ritter reaction.** Trifluoroacetic acid (40 ml) and acetonitrile (20 ml) were mixed and 1 g (0.0024 mol) of 4-dehydroxy-ε-rhodomycinone was added to the solution. The mixture was stirred at 60 °C for 3 h and subsequently concentrated on rotary evaporator to obtain a viscous residue. The residue was dissolved in chloroform (80 ml) and washed with water (100 ml). The water phase was extracted twice with chloroform (both 20 ml), and the combined chloroform extracts washed with saturated aqueous sodium bicarbonate solution (100 ml), water (100 ml) and saturated sodium chloride solution (80 ml). The organic phase was dried over sodium sulfate, and the solvent removed *in vacuo.* The dry residue was purified by silica gel chromatography (eluent: methanol/chloroform 1:40) and 948 mg of the cyclic Ritter reaction product was obtained as orange crystalline solid. A yield of 90% was achieved.

**Step 2: Hydrolysis of the Ritter reaction product.** 100 mg of Ritter reaction product from example 1 was dissolved in mixture of 1 ml of conc. sulfuric acid, 2 ml of water and 9 ml of tetrabydrofurane. The reaction mixture was heated under reflux for 5 days. The reaction mixture was extracted 2 times with 10 ml diethyl ether to remove side products. After that, reaction mixture was neutralized with 0.5 M sodium hydroxide to pH ∼5, and then with saturated sodium bicarbonate solution to pH 7.8 and extracted with chloroform (2 times 20 ml). Combined chloroform fractions were washed with water (30 ml) and saturated sodium chloride solution (20 ml). The organic phase was dried over sodium sulfate and solvent was evaporated and remaining solid was dried *in vacuo.* The yield was 39 mg of pure 9-amino-4-dehydroxy-ε-rhodomycinone.

### Example 2

In daunomycinone (R₁ = H, R₂ = OCH₃, R₃ = R = R₈ = OH, R₅ =H, R₆ = COCH₃, R₇ =OH; Formula IV above), the R₇ is transformed into -NH₂ by Ritter amination.

**Step 1: Ritter reaction.** Trifluoroacetic acid (100 ml) and acetonitrile (40 ml) were mixed and 2 g (0.005 mol) of daunomycinone was added to the solution. The mixture was stirred at 60 °C for 3 h and subsequently concentrated *in vacuo.* The resultant solid was dissolved in chloroform (100ml) and washed with water (100 ml). The water phase was extracted twice with chloroform (150 and 120 ml), and the combined chloroform extracts washed with saturated aqueous sodium bicarbonate solution (100 ml). The organic phase was dried over sodium sulfate, and the solvent removed *in vacuo*. The dry residue was purified by silica gel chromatography (eluent: 2.5% methanol/chloroform) and 900 mg of the Ritter intermediate (imidazoline cycle) was obtained.

**Step 2a. Ritter intermediate hydrolysis. Experiment 1**. 450 mg of the Ritter intermediate of example 2 was dissolved in a mixture of tetrahydrofurane (33.75 ml), concentrated sulfuric acid (3.75 ml) and water (7.5 ml) and heated at 60 °C for 30 h. The reaction mixture was extracted with ether (2 x 200 ml) and ethyl acetate (4 x 100 ml) and the pH was taken to 3.5 with 0.5 M sodium hydroxide solution. The solution was extracted with chloroform (200 ml). Saturated aqueous sodium bicarbonate solution was added to obtain the pH of 7.5, followed by extraction with chloroform (200 ml). The final chloroform extract was washed with water (200 ml) and brine (400 ml), which were subsequently back-extracted with chloroform (200 ml). The chloroform phases were combined and dried over sodium sulfate. The solution was filtered, the solvent removed under reduced pressure and the product dried *in vacuo.* The dry residue (200mg) was dissolved in chloroform (5 ml), followed by the addition of 3M methanolic hydrochloric acid (3 equivalents) and ether (5 ml) and left at -20 °C overnight. The solution was filtered, dried *in vacuo* and 145 mg of the 9-deoxy-9-amino-daunomycinone hydrochloride obtained.

**Step 2b. Ritter intermediate hydrolysis. Experiment 2**. 450 mg of the Ritter intermediate of example 2 was dissolved in 3 N sulfuric acid (45 ml) and heated at 60 °C for 30 h. The after-treatments were carried out in the same manner as in experiment 1 2. 200 mg of the obtained 9-deoxy-9-amino-daunomycinone was dissolved in chloroform (5 ml), followed by the addition of 3M methanolic hydrochloric acid (3 equivalents) and ether (5 ml) and left at -20 °C overnight. The solution was filtered, dried *in vacuo* and 115 mg of the 9-deoxy-9-amino-daunomycinone hydrochloride obtained.

**Step 2c. Ritter intermediate hydrolysis, experiment 3.** 700 mg of the Ritter intermediate of example 2 was treated as in experiment 1 using a mixture of 525ml of tetrahydrofurane, 116.7 ml of concentrated sulfuric acid and 58.3 ml of water and heating at 60 °C for 7 h. 600 mg of the 9-deoxy-9-amino-daunomycinone was obtained and dissolved in chloroform (10 ml). After the addition of 3 M methanolic hydrochloric acid (2 equivalents) and ether (10 ml), the solution was filtered, dried *in vacuo* and 400 mg of the 9-deoxy-9-amino-daunomycinone hydrochloride was obtained

### Example 3

For preparation of aglycon of amrubicine, daunomycinone (R₁ = H, R₂ = OCH₃, R₃ = R₄ = R₈ = OH, R₅ =H, R₆ = COCH₃, R₇ =OH; Formula V above), first a demethoxylation of R₂ = OCH₃ into R₂ = OH is performed as known in the state of the art, followed by dehydroxylation of R₂ = OH into R₂ = H by methods described in (WO 01/87814 and US 2006/0047108). The resulting 4-demethoxy daunomycinone (idamycinone, formula VI] is then converted to aglycon of amrubicine as follows.

**Step Ritter reaction.** Trifluoroacetic acid (32 ml) and acetonitrile (32 ml) were mixed and warmed to 55 °C and 456 mg (1.24 mmol) of 4-demethoxydaunomycinone was added to the solution. The mixture was stirred at 60 °C for 45 min and subsequently concentrated on rotary evaporator to obtain a viscous residue. The residue was dissolved in chloroform (75ml) and washed with 1.6% sodium bicarbonate solution (75 ml) and two times with water (both 75 ml). The organic phase was dried over sodium sulfate, and the solvent removed *in vacuo.* The dry residue was purified by silica gel chromatography (eluent: first methanol/chloroform 1:50, then methanol:chloroform 1:10). 212 mg of the cyclic Ritter reaction product was obtained as orange crystalline solid. A yield of 44% was achieved.

**Step 2. Ritter intermediate hydrolysis.** 10 mg of the cyclic Ritter reaction product from Step 1 was dissolved in 3N sulfuric acid and heated at 80 °C for 3 days. The mixture was neutralized by saturated NaHCO₃ solution and the product was extracted by 3x10 mL dichloromethane. The extracts were combined and evaporated in vacuo. The crude product was purified by preparative TLC. The molecular weight of product (366,09732) was consistent with the HRMS calculated for aglycone of amrubicine (366,09831 in negative mode).

Glycosylation may be achieved through using chemical coupling (R₁ = R₂= H, R₃ = R₄ = R₈ = OH, R₇ =NH₂, R₅ = H, R₆ = COCH₃) with 6-bromotetrahydro-2*H-*pyran-3,4-diyl diacetate or other protected derivatives of 2-deoxy-D-ribose.

In addition to the starting compounds described above in Examples 1 and 2, the present invention also contemplates reaction schemes that employ aklavinone as a starting compound. In general, the starting compound will determine the structure of the final product. As such, the use of different starting compounds within the context of the present invention will generate sets of novel compounds that would, in turn, serve as leads of new biologically active compounds.

Nothing in the above description is meant to limit the present invention to any specific materials, geometry, or orientation of atoms. Many part/orientation substitutions are contemplated within the scope of the present invention and will be apparent to those skilled in the art. The embodiments described herein were presented by way of example only and should not be used to limit the scope of the invention.

Although the invention has been described in terms of particular embodiments in an application, one of ordinary skill in the art, in light of the teachings herein, can generate additional embodiments and modifications without departing from the spirit of, or exceeding the scope of, the claimed invention. Accordingly, it is understood that the drawings and the descriptions herein are proffered only to facilitate comprehension of the invention and should not be construed to limit the scope thereof.

## Claims

1. A method of producing amrubicin (Formula I) 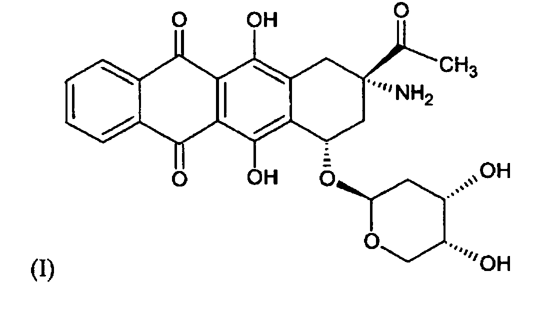 by modifying a natural or semisynthetic anthracycline aglycone (Formula II) 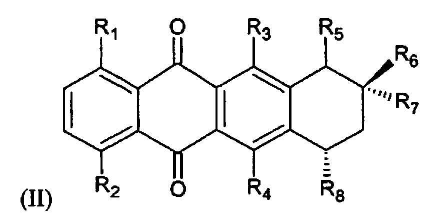 wherein R₁, R₂, R₃, R₄, and R₈ each are -H, -OH, or alkoxy; R₅ is -H, alkyl, or alkoxycarbonyl; R₆ is -H, acyl or alkyl; and R₇ is -OH;
chemically or by biosynthetic methods to aglycon of amrubicine (Formula III) 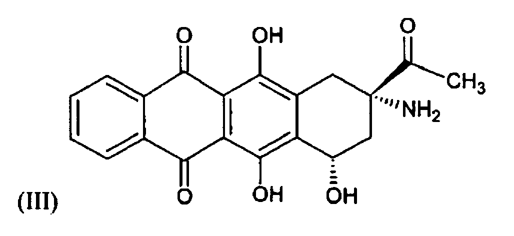 followed by glycosylation of the aglycon of amrubicine to form amrubicin.

2. Method of claim 1, where an anthracycline intermediate with Formula VI (idarubicinone, 4-demethoxydaunomycinone) 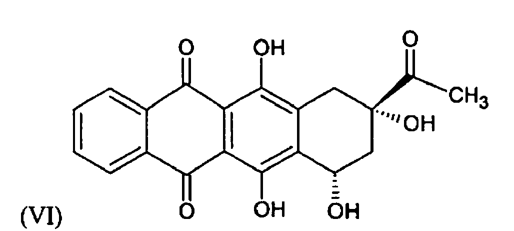 is converted to aglycon of amrubicine (Formula III) by chemical modification of R₇ = -OH to R₇ = -NH₂.

3. Method of claim 2, where said chemical modification of R₇ is done by Ritter reaction with a suitable nitrile with formule R₉-CN forming a cyclic intermediate with Formula VIII 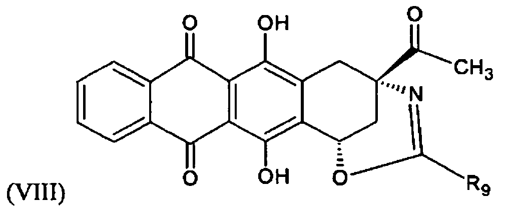 or a non-cyclic intermediate with Formula VIIIa 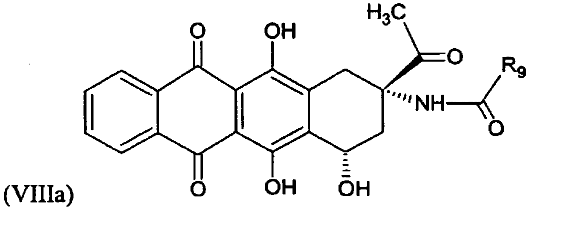 , where R₉ is alkyl, halogenated alkyl, hydrogen or aryl group, followed by hydrolysis of said intermediate under acidic conditions to form aglycon of amrubicine (Formula III).

4. Method for production of aglycon of amrubicin, where 4-demethoxydaunomycinone (formula VI) is put into Ritter reaction with mixture of equal volumes of acetonitrile and trifluoroacetic acid at 60 °C, followed by hydrolysis of formed cyclic intermediate with formula VIII (where R₉ = -CH₃) in 3N aqueous solution of sulfuric acid at 80°C to form aglycon of amrubicine with formula III.

5. Method of claim 3, where Ritter reaction of anthracycline with Formula VI is carried out but reacting said anthracycline with acetonitrile (where R₉ = -CH₃) in the presence of a suitable acid.

6. Method of claim 5, where the suitable acid used for Ritter reaction is trifluoroacetic acid.

7. Method of claim 5, where the suitable acid used for Ritter reaction is sulfuric acid.

8. A method of chemical modification of an anthracycline aglycone with Formula II, where R₇ = OH and R₈ = OH to respective anthracycline aglycone, where R₇ = NH₂ and R₈ = OH.

9. Method of claim 8, where said chemical modification is performed by Ritter reaction with an alkyl- or halogenated alkylnitrile in the presence of a suitable acid.

10. Method of claim 8, where the anthracycline aglycone being modified is daunomycinone.

11. Method of claim 8, where the anthracycline being modified is 4-dehydroxy-ε-rhodomycinone.

12. Method of claim 3, where said aglycon of amrubicin with formula III, is further glycosylated to form amrubicin with formula I.

## Patentansprüche

1. Herstellungsverfahren für Amrubicin (Formel I) 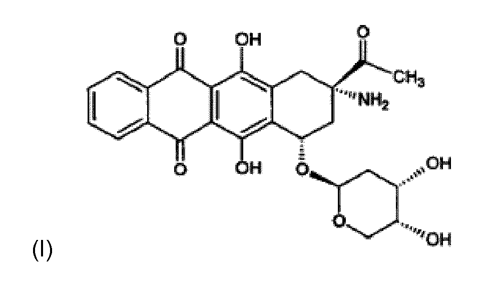 durch Umwandlung eines natürlichen oder halbsynthetischen Anthracyclin-Aglycons (Formel II) 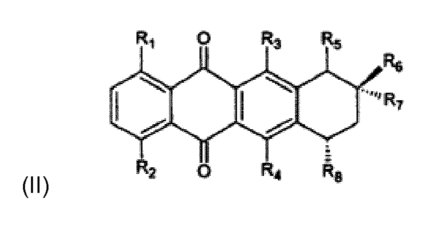 worin R₁, R₂, R₃, R₄ und R₈ jeweils -H, -OH oder Alkoxy sind; R₅ -H, Alkyl oder Alkoxycarbonyl ist; R₆ -H, Acyl oder Alkyl ist; und R₇ -OH ist;
chemisch oder mit biosynthetischen Verfahren zu einem Aglycon von Amrubicin (Formel III) 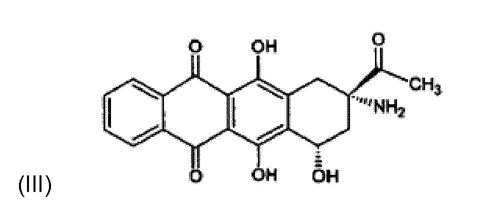 gefolgt von einer Glycosylierung des Aglycons von Amrubicin zum Bilden von Amrubicin.

2. Verfahren gemäß Anspruch 1, wobei ein Anthracyclin-Zwischenprodukt der Formel VI (Idarubicinon, 4-Demethoxydaunomycinon) 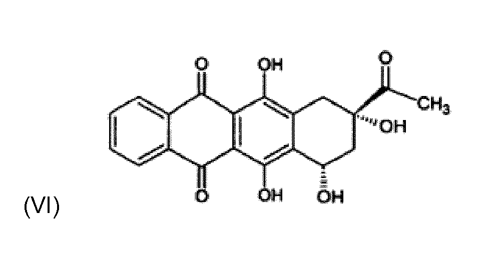 zu Anglycon von Amrubicin (Formel III) umgewandelt wird durch chemische Umwandlung von R₇ = -OH zu R₇ = -NH₂.

3. Verfahren gemäß Anspruch 2, wobei die chemische Umwandlung von R₇ durch eine Ritter-Reaktion erfolgt, mit einem geeigneten Nitril der Formel R₉-CN zum Bilden eines cyclischen Zwischenprodukts der Formel VIII 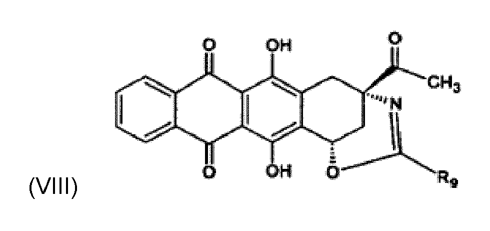 oder eines nicht cyclischen Zwischenprodukts der Formel VIIIa 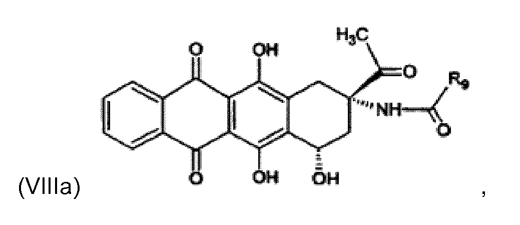 worin R₉ Alkyl, halogeniertes Alkyl, Wasserstoff oder Arylgruppe ist, gefolgt von einer Hydrolyse des Zwischenprodukts unter sauren Bedingungen zum Bilden von Aglycon von Amrubicin (Formel III).

4. Herstellungsverfahren für Aglycon von Amrubicin, wobei 4-Demethoxydaunomycinon (Formel VI) einer Ritter-Reaktion mit einem Gemisch gleicher Volumen Acetonitril und Trifluoroessigsäure bei 60°C zugeführt wird, gefolgt von einer Hydrolyse von gebildetem cyclischen Zwischenprodukt der Formel VIII (wobei R₉ = -CH₃) in 3N-wässriger Schwefelsäurelösung bei 80°C zum Bilden von Aglycon von Amrubicin der Formel III.

5. Verfahren gemäß Anspruch 3, wobei die Ritter-Reaktion von Anthracyclin der Formel VI durchgeführt wird durch Reagieren des Anthracyclins mit Acetonitril (worin R₉ = -CH₃) in der Anwesenheit einer geeigneten Säure.

6. Verfahren gemäß Anspruch 5, wobei die in der Ritter-Reaktion verwendete geeignete Säure Trifluoroessigsäure ist.

7. Verfahren gemäß Anspruch 5, wobei die in der Ritter-Reaktion verwendete geeignete Säure Schwefelsäure ist.

8. Verfahren zur chemischen Umwandlung von Anthracyclin-Aglycon der Formel II, wobei R₇ = OH und R₈ = OH zum entsprechenden Anthracyclin-Aglycon, woein R₇ = -NH₂ und R₈ = OH.

9. Verfahren gemäß Anspruch 8, wobei die chemische Umwandlung durch Ritter-Reaktion mit einem Alkyl- oder einem halogenierten Alkylnitril in der Anwesenheit einer geeigneten Säure ausgeführt wird.

10. Verfahren gemäß Anspruch 8, wobei das Anthracyclin-Aglycon, das umgewandelt wird, Daunomycinon ist.

11. Verfahren gemäß Anspruch 8, wobei das Anthracyclin, das umgewandelt wird, 4-Dehydroxy-ε-rhodomycinon ist.

12. Verfahren gemäß Anspruch 3, wobei das Aglycon von Amrubicin der Formel III weiter glycosyliert wird zum Bilden von Amrubicin der Formel I.

## Revendications

1. Un procédé de production d'amrubicine (Formule I) 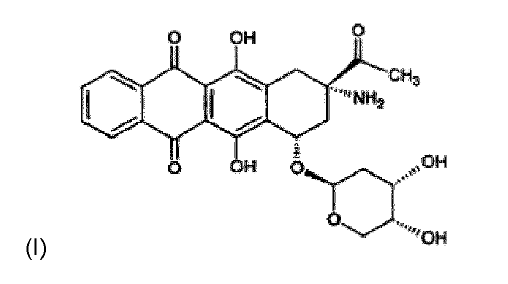 par la modification d'un aglycone d'anthracycline naturel ou semi-synthétique (Formule II) 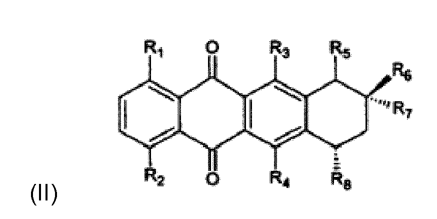 où R₁, R₂, R₃, R₄ et R₈ sont chacun -H, -OH ou alkoxy ; R₅ est -H, alkyle ou alkoxycarbonyl, R₆ est -H, acyle ou alkyle et R₇, est -OH,
chimiquement ou par des procédés biosynthétiques en aglycone d'amrubicine (Formule III) 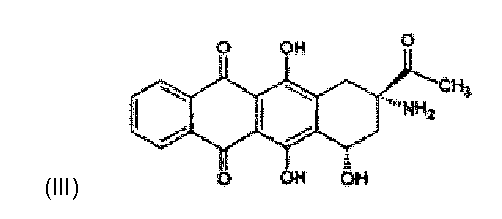 suivie par une glycosylation de l'aglycone d'amrubicine de façon à former l'amrubicine.

2. Le procédé selon la revendication 1, où un intermédiaire d'anthracycline de la Formule VI (idarubicinone, 4-déméthoxydaunomycinone) 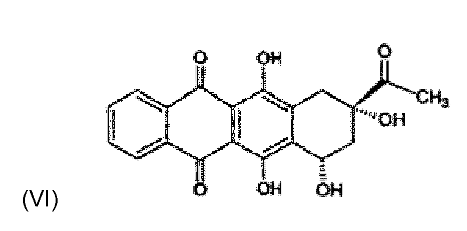 est converti en aglycone d'amrubicine (Formule III) par la modification chimique de R₇ = -OH en R₇ = -NH₂.

3. Le procédé selon la revendication 2, où ladite modification chimique de R₇ est réalisée par une réaction de Ritter avec un nitrile adapté de la formule R₉-CN formant un intermédiaire cyclique de la Formule VIII 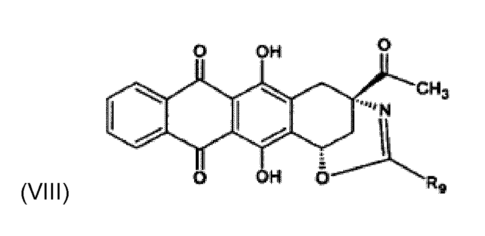 ou un intermédiaire non cyclique de la Formule VIIIa 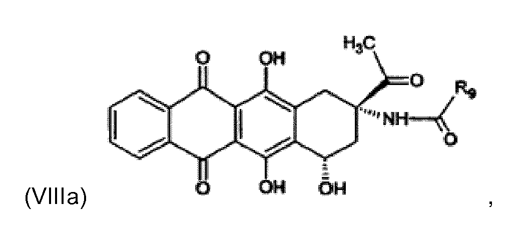 où R₉ est alkyle, alkyle halogéné, hydrogène ou un groupe aryle, suivie par une hydrolyse dudit intermédiaire dans des conditions acides de façon à former un aglycone d'amrubicine (Formule III).

4. Un procédé de production d'aglycone d'amrubicine, où 4-déméthoxydaunomycinone (formule VI) est mis en réaction de Ritter avec un mélange de volumes égaux d'acétonitrile et d'acide trifluoroacétique à 60°C, suivie par une hydrolyse de l'intermédiaire cyclique formé de la formule VIII (où R₉ = -CH₃) dans une solution aqueuse 3N d'acide sulfurique à 80°C de façon à former un aglycone d'amrubicine de la formule III.

5. Le procédé selon la revendication 3, où une réaction de Ritter d'anthracycline de la Formule VI est réalisée par une réaction de ladite anthracycline avec acétonitrile (où R₉ = -CH₃) en présence d'un acide adapté.

6. Le procédé selon la revendication 5, où l'acide adapté utilisé pour la réaction de Ritter est de l'acide trifluoroacétique.

7. Le procédé selon la revendication 5, où l'acide adapté utilisé pour la réaction de Ritter est de l'acide sulfurique.

8. Un procédé de modification chimique d'un aglycone d'anthracycline de la Formule II, où R₇ = OH et R₈ = OH vers un aglycone d'anthracycline respectif, où R₇ = NH₂ et R₈ = OH.

9. Le procédé selon la revendication 8, où ladite modification chimique est réalisée par une réaction de Ritter avec un alkyle ou un alkylenitrile halogéné en présence d'un acide adapté.

10. Le procédé selon la revendication 8, où l'aglycone d'anthracycline qui est modifié est daunomycinone.

11. Le procédé selon la revendication 8, où l'anthracycline qui est modifiée est 4-déhydroxy-ε-rhodomycinone.

12. Le procédé selon la revendication 3, où ledit aglycone d'amrubicine de la formule III est encore glycosylé de façon à former l'amrubicine de la formule I.
